# EUROPÄISCHE PATENTSCHRIFT

(11) **EP 2 943 135 B1**
(45) Veröffentlichungstag und Bekanntmachung des Hinweises auf die Patenterteilung: **05.04.2017**
(21) Anmeldenummer: 13817902.3
(22) Anmeldetag: 12.12.2013
(51) Int. Cl.: A61B 17/12

(54) **LIGATORSYSTEM MIT ZWISCHENRING ZWISCHEN DEN RINGBÄNDERN**
LIGATURE SYSTEM WITH INTERMEDIATE RING BETWEEN THE RING BANDS
SYSTÈME DE LIGATEUR POURVU D'UNE BAGUE INTERMÉDIAIRE DISPOSÉE ENTRE LES LIGAMENTS ANNULAIRES

(30) Priorität: 08.01.2013 DE 202013100076 U
(43) Veröffentlichungstag der Anmeldung: 18.11.2015
(73) Patentinhaber: Maurus, Michael, 87509 Immenstadt (DE)
(72) Erfinder: Maurus, Michael, 87509 Immenstadt (DE)
(74) Vertreter: Misselhorn, Hein-Martin
(86) Internationale Anmeldenummer: PCT/EP2013/076390
(87) Internationale Veröffentlichungsnummer: WO 2014/108270

(56) Entgegenhaltungen:
- DE-A1- 19 834 263
- US-A- 6 059 719
- US-A1- 2006 259 041
- US-A1- 2011 106 116

## Beschreibung

Die Erfindung betrifft ein Ligatorsystem gemäß dem Oberbegriff des Anspruchs 1 sowie einen Zwischenring für ein solches Ligatorsystem.

Unter einer Band-Ligatur versteht man im Allgemeinen das Applizieren eines elastisch aufgeweiteten Ringbandes an einen durch das Ringband in seinem entspannten Zustand abzubindenden anatomischen Bereich eines Lebewesens.

Ein Ligatorsystem besteht gemeinhin aus einem Ligator genannten medizinischen Gerät, mit welchem die zur Ligatur dienenden Ringbänder über den abzubindenden anatomischen Bereich gestülpt werden, und den zugehörigen Ringbändern.

Zu diesem Zweck besitzt der Ligator typischerweise ein Halterohr, auf dessen Außenumfang das Ringband aufgesetzt wird. Hierbei wird das Ringband gedehnt, so dass sich der Innendurchmesser des Ringbandes stark vergrößert. Das Halterohr wird über den zu behandelnden anatomischen Bereich gestülpt und das abzubindende Gewebe wird in das Halterohr verbracht, beispielsweise, indem es von diesem eingesaugt wird. Daraufhin wird das Ringband so weit zum distalen Ende des Halterohrs verschoben, dass es von dem Halterohr abspringt; man spricht hier plakativ ausgedrückt von einem "Abfeuern" des Ringbandes, obwohl der Vorgang rein mechanischer Natur ist. Sobald das Ringband vom Halterohr abgesprungen ist, zieht es sich schlagartig elastisch zusammen und bindet den so behandelten anatomischen Bereich ab.

Die US 2006/0259 A1 offenbart eine endoskopische Vorrichtung mit Zwischenelementen, welche mithilfe von Zugmitteln radial verformt werden, so dass das so verformte Zwischenelement dem Abstreifen des nächsten Bandes nicht länger im Wege steht.

Die US 2011/0106116 A1 offenbart eine Vorrichtung zum Verschließen von Gewebeöffnungen. Dabei wird das Band zum Verschließen von Gewebe mit einem Betätigungshebel kontrolliert gelöst und gesetzt. Diese Bänder weisen keine Zwischenelemente auf, auf dem Halterohr sind integral mit ihm verbundene Rillen angebracht. Die DE 19834263 A1 offenbart ein Ligatorsystem entsprechend den Merkmalen des Oberbegriffs des unabhängigen Anspruchs 1.

Es ist wünschenswert, mehrere Ringbänder hintereinander auf das Halterohr aufsetzen zu können, um sie an unterschiedlichen zu behandelnden Stellen kontrolliert nacheinander "abfeuern" und somit an unterschiedlichen Stellen eine Ligatur herstellen zu können, ohne zwischendurch wieder von neuem ein weiteres Ringband auf das Halterohr des Ligators aufsetzen zu müssen. Hierbei tritt in der Praxis allerdings immer wieder das Problem auf, dass unbeabsichtigt zwei Ringbänder gleichzeitig von der Haltehülse abgestreift werden, falls man versucht, die Ringbänder hintereinander auf dem Rohr zu platzieren, was dazu führt, dass ein und derselbe zu behandelnde Bereich durch zwei Ringbänder abgebunden wird. Dies ist nicht nur unökonomisch und kontraproduktiv im Hinblick auf den gewünschten, schnellen Arbeitsfortschritt, sondern birgt aus verschiedenen Gründen auch die Gefahr medizinischer Komplikationen.

Es ist daher die Aufgabe der vorliegenden Erfindung, ein Ligatorsystem zu schaffen, welches eine erhöhte Sicherheit gegen das unbeabsichtigte Abfeuern von zwei Ringbändern zur gleichen Zeit bietet.

Diese Aufgabe wird mit den Mitteln des Anspruchs 1 gelöst.

Das erfindungsgemäße Ligatorsystem zeichnet sich dadurch aus, dass zwei hintereinander auf das Halterohr des Ligators aufgeschobene Ringbänder nicht unmittelbar gegeneinander anliegen, sondern voneinander durch einen dazwischen aufgeschobenen Zwischenring getrennt sind. Dieser Zwischenring hält die beiden Ringbänder, in Richtung der Längsachse des Halterohrs gesehen, auf Abstand voneinander. Hierdurch wird sichergestellt, dass das nächstfolgende Ringband auch nach dem Abfeuern des vorhergehenden Ringbandes nach wie vor noch einen mehr als nur unerheblichen Abstand vom distalen Ende des Halterohrs einnimmt und daher dem distalen Ende des Halterohrs auch unter ungünstigen Umständen nicht so nahe kommt, dass es unbeabsichtigt von dem Halterohr abspringt. Dies vereinfacht das Abfeuern eines Ringbandes erheblich, da es nicht mehr notwendig ist, den Abzug extrem feinfühlig zu betätigen, um auf diese Art und Weise zu gewährleisten, dass sich durch das Vorschieben der Ringbänder entlang der Längsachse L des Halteabschnitts zum Zwecke des Abfeuerns des äußersten Ringbandes auch tatsächlich nur dieses Ringband von der Haltehülse des Ligators löst.

Die Erfindung ermöglicht es, dass sich der behandelnde Arzt darauf beschränkt, den Ligator nach dem Abfeuern eines Ringbandes kurz zurückzuziehen, um den Zwischenring abzunehmen. Dann kann sofort mit dem Legen eines weiteren Ringbandes fortgefahren werden - ohne erst umständlich ein neues Ringband auf die Haltehülse des Ligators aufziehen zu müssen. Dies erleichtert insbesondere die Ligatur zur Behandlung von Hämorrhoidalleiden, aber auch von inneren und äußeren Schleimhautvorfällen, von zu stillenden Blutungen (z.B. im Magen-Darmtrakt bzw. auch das Abbinden von anderem zu entfernenden Gewebe im Bereich des Magen-Darmtraktes). Denn das Aufziehen eines neuen, im Zuge des Aufziehens erst noch aufzudehnenden Ringbandes auf die Haltehülse eines bereits zum Einsatz gekommenen Ligators ist unter hygienischen Gesichtspunkten wesentlich schwieriger zu bewerkstelligen als das bloße Abnehmen eines nicht mehr benötigten Zwischenrings. Auch kann dadurch der personelle sowie der apparative Aufwand reduziert werden und es können Kosten auch bzgl. der sonst notwendigen Sterilisierungsmaßnahmen (Zeit und Material sowie Chemikalien/Desinfektionsmittel/Verpackungsmaterial) eingespart werden.

Vorzugsweise besteht der oder bestehen die Zwischenringe aus einem anderen Material als die Ringbänder. Für die nicht längere Zeit mit dem Körper in Kontakt kommenden Zwischenringe kann ein preisgünstigeres Material verwendet werden und das Material der Zwischenringe kann auf diese Art und Weise an die Funktion der Zwischenringe angepasst werden, die wesentlich weniger dehnbar sein müssen als die Ringbänder.

Es ist zweckmäßig, wenn der Zwischenring aus einem Material besteht, dessen Federhärte D um mindestens den Faktor 5 und idealerweise sogar mindestens um den Faktor 7,5 größer ist als die Federhärte des Materials, aus denen die Ringbänder sind. Dadurch wird sichergestellt, dass der Zwischenring eine wesentlich geringere Tendenz hat, unbeabsichtigt von dem Halterohr des Ligators abzuspringen, als es die Ringbänder haben. Außerdem wird dadurch gewährleistet, dass der Zwischenring den vorliegenden Gummiring schieben kann und von dem nachfolgenden Gummiring nicht unerwünschterweise zusammengepresst wird. Denkbar wären auch Zwischenringe aus Metall, welche ggf. nach der Benutzung hygienisch aufbereitet würden, um erneut verwendet werden zu können.

Idealerweise ist die Breite des Zwischenrings 12 in Richtung parallel zur Längsachse L des Halterohrs größer als die Breite oder der Durchmesser der Ringbänder parallel zur Längsachse L. Ein Verhältnis von mindestens 1,5:1 oder sogar besser noch mindestens 2:1 zu Gunsten der Breite des Zwischenrings 12 hat sich bewährt. Auch dies trägt dazu bei, dass der Zwischenring nicht unbeabsichtigt vom Halteabschnitt des Ligators abspringt oder abgezogen wird. Aufgrund seiner größeren Breite findet der Zwischenring sicheren Halt auf dem Halteabschnitt des Ligators.

Zweckmäßigerweise liegt der Zwischenring nur lokal gegen die Oberfläche des Halterohrs an. Idealerweise wird dies dadurch bewerkstelligt, dass der Zwischenring an seiner inneren Umfangsfläche mindestens drei sich in radial einwärtiger Richtung erstreckende Vorsprünge oder Nasen besitzt, mit denen er gegen die Umfangs-Oberfläche des Halterohrs anliegt. Auf diese Art und Weise ist es einfacher, auch bei Verwendung eines vergleichsweise harten Materials für den Zwischenring, eine definierte Vorspannung des Zwischenrings gegenüber der Oberfläche des Halterohrs zu erreichen als bei einem flächigen Anliegen des gesamten Innenumfangs des Zwischenrings. Auch kann dadurch der Reibungswiderstand und dadurch der Kraftaufwand bei der Anwendung reduziert werden. Somit können für den Zwischenring größere Maß-Toleranzen zugelassen werden, was nicht zuletzt dem Bedürfnis entgegenkommt, den Zwischenring als ein möglichst preisgünstiges Wegbauteil auszuführen, das nach der einmaligen Anwendung verworfen wird.

Vorzugsweise ist der Zwischenring derart an das Halterohr des Ligators angepasst, dass er beim Aufschieben auf das Halterohr eine elastische Verformung erfährt und dadurch derart vorgespannt gegen die Oberfläche des Halterohrs anliegt, dass er nur unter Überwindung einer mehr als nur unwesentlichen Reibung auf dem Halterohr verschoben werden kann. Auf diese Art und Weise kann die Reibung zwischen der Außenoberfläche des Halterohrs und dem Zwischenring ohne weiteres so eingestellt werden, dass der Zwischenring nicht unbeabsichtigt und vorzeitig verloren geht.

Idealerweise ist der kleinste Innendurchmesser des Zwischenrings um mindestens 0,1 mm und idealerweise um mindestens 0,2 mm kleiner als der Außendurchmesser des Halterohrs.

Schutz wird auch für den Zwischenring als solchen beansprucht, also für einen Zwischenring, der zum auf Abstand halten zweier oder mehrerer hintereinander auf ein Halterohr eines Ligators aufgezogener Ringbänder dient, wobei der Innendurchmesser des Zwischenrings an den Außendurchmesser des Halterohrs des Ligators angepasst ist, mit dem der Zwischenring bestimmungsgemäß verwendet wird.

Der Vorteil des erfindungsgemäßen Zwischenrings liegt nicht zuletzt darin, dass er bei entsprechend angepasster Dimensionierung dazu verwendet werden kann, um ein bereits bestehendes und bisher dem Stand der Technik entsprechendes Ligatorsystem so aufzurüsten, dass es erfindungsgemäß ist. Denn an den meisten Ligatoren steht ein freier Halteabschnitt zur Verfügung, der in Richtung seiner Längsachse gesehen breiter ist als der Durchmesser eines Ringbandes, was es möglich macht, mindestens zwei Ringbänder und einen dazwischen liegenden Zwischenring auf den Halteabschnitt des bestehenden Ligators aufzuziehen.

Dementsprechend wird auch für die Verwendung des erfindungsgemäßen Zwischenrings zur Nachrüstung von bereits bestehenden Ligatoren mit unterschiedlicher Länge und unterschiedlichen Durchmessern Schutz beansprucht.

Darüber hinaus wird auch für ein Verfahren zum Setzen eines Ringbandes selbstständiger Schutz beansprucht, welches wie folgt ausgeführt wird:
Zur Durchführung des Verfahrens wird ein Ligator verwendet, auf dessen Halteabschnitt in Richtung der Längsachse des Halteabschnitts gesehen mindestens zwei Ringbänder hintereinander aufgezogen sind, die voneinander durch einen Zwischenring getrennt werden. Um an einer ersten zu behandelnden Stelle eine Ligatur herzustellen, wird zunächst nur ein Ringband abgefeuert. Danach wird der Ligator zurückgezogen und es wird der Zwischenring abgenommen. Daraufhin wird der Ligator an eine zweite zu behandelnde Stelle geführt, an der nun mit Hilfe des noch auf dem Halteabschnitt des Ligators befindlichen zweiten Ringbandes eine weitere Ligatur hergestellt wird. Ggf. kann danach durch einen weiteren Zwischenring getrennt ein dritter oder vierter Gummiring aufgezogen und separat abgefeuert werden.

Darüber hinaus wird unabhängiger Schutz für die Verwendung mindestens eines losen, d. h. komplett (werkzeuglos) vom Halteabschnitt abnehmbaren Zwischenrings zum Applizieren aufgeweiteter Ligatur-Ringe ohne zwischenzeitliches Nachladen an einen durch den jeweiligen Ligatur-Ring in seinem entspannten Zustand abzubindenden anatomischen Bereich eines Lebewesens mit Hilfe eines Ligators, der ein Halterohr umfasst, welches den jeweils abzubindenden anatomischen Bereich in seinem Inneren aufnimmt und mit seiner Außenfläche mehrere (vorzugsweise in einer Flucht hintereinander) angeordnete Ligatur-Ringe aufgespannt hält, beansprucht, wobei sich die beanspruchte Verwendung dadurch auszeichnet, dass der Zwischenring zum auf Abstand halten zweier hintereinander vorzugsweise in einer Flucht auf ein Halterohr eines Ligators aufgezogener Ligatur-Ringe zum Einsatz kommt, wobei der Innendurchmesser des Zwischenrings an den Außendurchmesser des Halterohrs des Ligators angepasst ist, mit dem der Zwischenring bestimmungsgemäß verwendet wird, so, dass der Zwischenring vorzugsweise durch Reibungskräfte auf dem Halterohr gehalten wird, die groß genug sind, um zu verhindern, dass beim willkürlichen Abfeuern eines Ligatur-Rings unwillkürlich ein weiterer Ligatur-Ring vom Haltering abspringt. Dabei wird der Zwischenring vorzugsweise so verwendet, dass er nach einmaligem Gebrauch verworfen wird.

Unter dem Begriff "zwischenzeitliches Nachladen" versteht man das Aufziehen eines weiteren Ligatur-Rings auf dem Halteabschnitt zwischen dem Abfeuern zweier Ligatur-Ringe.
Der insoweit entscheidende Punkt ist, dass das vollständige Abziehen eines Zwischenrings vom Halteabschnitt auch von der behandschuhten Hand des Operateurs nicht zu bewerkstelligen ist und daher keine wirkliche Unterbrechung des Arbeitsflusses bewirkt. Völlig anders ist das beim Aufziehen eines Ligatur-Rings, der im Regelfall mit Hilfe einer entsprechenden Vorrichtung stark gedehnt werden muss, um auf den Halteabschnitt aufgezogen werden zu können, was sich eben nicht mit der behandschuhten Hand "nebenbei" erledigen lässt, ohne Unterbrechung des Arbeitsflusses.

Weitere Wirkungsweisen, Vorteile und Ausgestaltungsmöglichkeiten der Erfindung ergeben sich aus der nachfolgenden Beschreibung eines Ausführungsbeispiels anhand der Figuren.
Die Figur 1 zeigt einen Ligator, wie er bei dem erfindungsgemäßen Ligatorsystem zum Einsatz kommt.
Die Figur 2 zeigt eine Vergrößerung des mit zwei Ringbändern und einem Zwischenring bestückten Halteabschnitts des von Figur 1 gezeigten Ligators.
Die Figur 3 zeigt das gleiche Ensemble wie die Figur 2, mit dem Unterschied, dass hier die beiden Ringbänder und der Zwischenring geschnitten dargestellt sind.
Die Figur 4 zeigt eine Detailansicht eines Ausführungsbeispiels des erfindungsgemäßen Zwischenrings.
Die Figur 5 zeigt schematisch eine einzelne Hämorrhoide in einem ebenfalls nur schematisch dargestellten Analkanal.
Die Figur 6 zeigt den über die von Figur 5 gezeigte Hämorrhoide gestülpten Halteabschnitt des Ligators unmittelbar vor dem Abfeuern des Ringbandes.
Die Figur 7 zeigt die mit den Mitteln der Figur 6 hergestellte, fertige Ligatur.

Die Ringband-Ligatur kann bekanntlich bei unterschiedlichen medizinischen Indikationen und an unterschiedlichen Stellen des menschlichen oder tierischen Körpers zur Anwendung kommen. Das erfindungsgemäße Ligatorsystem ist vom Grundsatz her für alle denkbaren Anwendungsfälle geeignet, wird aber idealerweise zur Behandlung von Hämorrhoidalleiden verwendet. Es entfaltet dort seine Stärken in besonderem Maß, da es die Handhabung des Ligators in diesem unter Hygienegesichtspunkten kritischen Bereich extrem erleichtert.

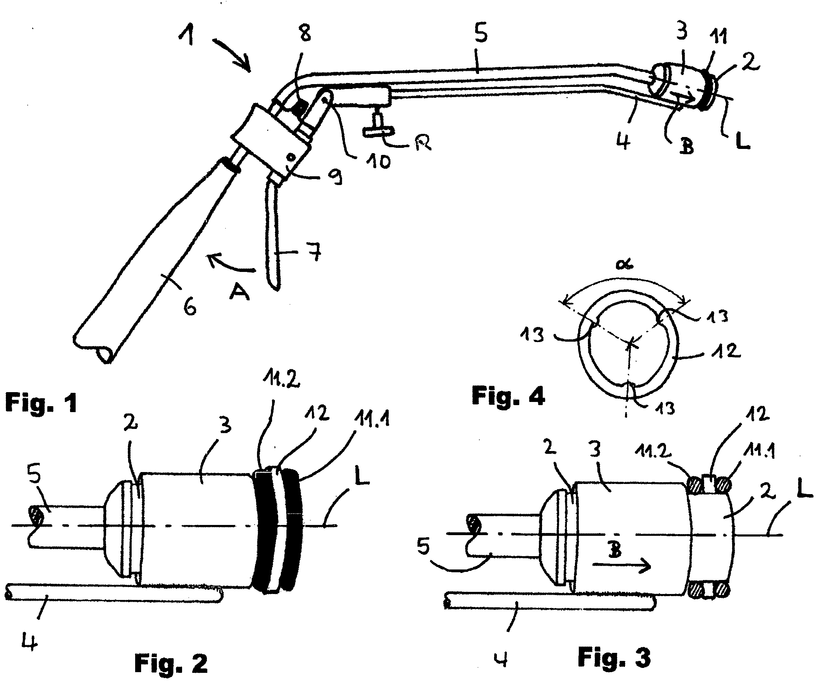

Die Figur 1 zeigt einen als solchen bekannten Ligator 1, der für das erfindungsgemäße Ligatorsystem zur Anwendung kommt. Es handelt sich hier um einen vollständig mit der Hand betätigten Ligator, bei dem auch das Abfeuern mit der Hand ausgelöst wird. Ein solcher Ligator ist besonders einfach aufgebaut und leicht zu sterilisieren, verhält sich aber unter Umständen beim Abfeuern kritischer als ein Ligator, bei dem das Abfeuern durch einen Elektroantrieb bewerkstelligt wird, der sehr exakt arbeiten und die Ringbänder bis auf Bruchteile eines Millimeters genau vorschieben kann.

Typischerweise besteht ein solcher Ligator aus einem Handstück 6, mit dem über ein Verbindungsstück 5 ein Halterohr 2 verbunden ist. Auf dem Halterohr 2 gleitet ein Vorschubrohr 3, welches über eine Vorschubstange 4 mit einem Abzug 7 verbunden ist. Zu diesem Zweck ist der Abzug 7 bei dem hier beschriebenen Ausführungsbeispiel in dem vom Verbindungsstück 5 getragenen Lagerblock 9 schwenkbar gelagert. Der Abzug 7 kann in Richtung des Pfeils A gezogen werden und überträgt dann seine Bewegung über das Verbindungslager 10 so auf die Vorschubstange 4, dass das Vorschubrohr 3 in Richtung des Pfeils B bewegt wird. Auf diese Art und Weise kann das Ringband 11, welches bislang von dem Halterohr 2 aufgespannt gehalten wird, das Halterohr 2 entlang, hin zu dessen distalem Ende, geschoben und dadurch "abgefeuert" werden - sobald das Ringband 11 das distale Ende des Halterohrs 2 erreicht hat, springt es unter dem Einfluss seiner Elastizität bzw. der von ihm durch das Aufspannen gespeicherten Energie von dem Halterohr 2 ab und zieht sich schlagartig zusammen, womit an der entsprechenden Stelle eine Ligatur hergestellt ist.

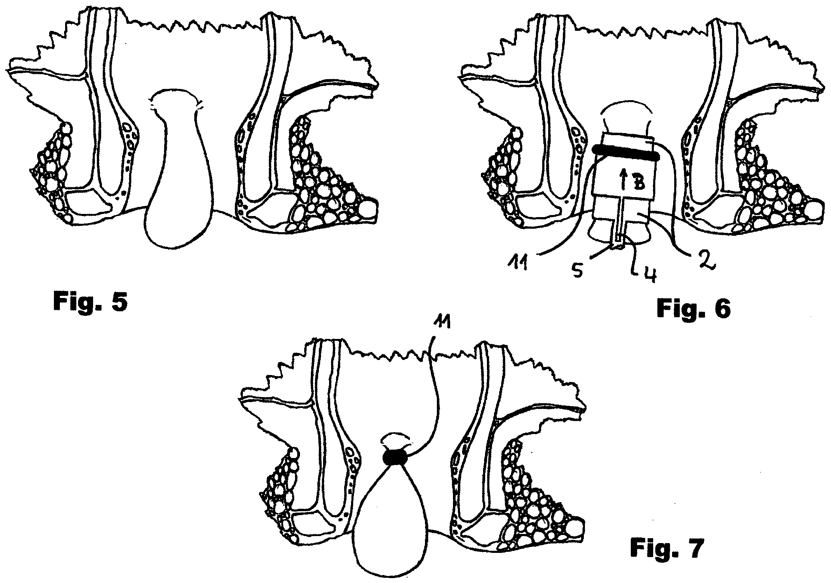

Der Halteabschnitt 2 kann an seinem proximalen Ende verschlossen sein und in das Verbindungsstück 5 übergehen, so wie das hier in den Figuren gezeigt ist. Alternativ kann das Halterohr 2 auch an seinem proximalen Ende offen sein, um auf diese Art und Weise das Ergreifen der zu behandelnden Stelle mittels einer Zange, mit Hilfe von Unterdruck (Luftsogsystem) oder dergleichen durch das Innere des Halterohrs 2 hindurch zu ermöglichen, so, wie das beispielsweise die Figur 6 andeutet.

Bemerkenswert an dem von Figur 1 gezeigten Ligator ist noch die mit dem Bezugszeichen R gekennzeichnete Klemmschraube. Löst man diese, lässt sich die Position einstellen, die das Vorschubrohr 3 auf dem Halterohr 2 einnimmt, bei unbetätigtem Abzug 7. Auf diese Art und Weise kann eingestellt werden, ob das Ligatorsystem zu Beginn der Behandlung mit einem oder mehreren Ringbändern und erfindungsgemäßen Zwischenringen bestückt wird. Somit kann gezielt derjenige Platz auf dem Halterohr 2 geschaffen werden, der benötigt wird, um den erfindungsgemäßen Zwischenring zusätzlich zu den Ringbändern auf das Halterohr 2 aufzuziehen.

Die Figuren 5 bis 7 zeigen das allgemein bekannte Prinzip der Ringband-Ligatur für den Spezialfall der Behandlung von Hämorrhoiden, um so das bevorzugte Anwendungsumfeld des erfindungsgemäßen Ligatorsystems zu veranschaulichen.

Zunächst ist der Blick auf die Figur 5 zu wenden. Schematisch ist hier der Analkanal mit einer einzelnen, hier natürlich nur grob skizzierten Hämorrhoide gezeigt.

Die Ringband-Ligatur wird in diesem Fall so durchgeführt, wie es die Figur 6 zeigt.

Mit Hilfe eines dem einschlägigen Fachmann bekannten und daher hier nicht zeichnerisch dargestellten Anoskops, Koloskops, Rektoskops oder Proktoskops wird der soeben beschriebene Ligator an die Hämorrhoide oder das Gewebe herangeführt, bis schließlich das Halterohr 2 des Ligators weitgehend über die Hämorrhoide gestülpt ist, so, dass sich der Ansatz der Hämorrhoide im Bereich der distalen, d. h. der dem Handstück 6 abgewandten Mündung des Halterohrs 2 befindet. Nun wird das Ringband 11 so "abgefeuert", wie das oben bereits beschrieben worden ist.

Die auf diese Art und Weise hergestellte Ligatur zeigt die Figur 7. Die Hämorrhoide ist durch das Ringband 11, welches sich wieder auf seinen ursprünglichen, sehr kleinen Durchmesser zusammengezogen hat, abgebunden. Sie wird im Regelfall nach einiger Zeit einschließlich des Ringbandes von alleine vom Körper abgestoßen.

Während die Figur 1 ein Ligatorsystem zeigt, bei dem auf den Ligator nur ein einziges Ringband aufgezogen ist, zeigen die Figuren 2, 3 und 4, worauf es bei der Erfindung ankommt.

Die Figur 2 zeigt einen Teil des Ligators gemäß Figur 1, nämlich den Teil des Ligators im Bereich des Halterohrs 2.

Gut zu erkennen ist hier der letzte Abschnitt des Verbindungsstücks 5, welches in das Halterohr 2 übergeht. Ebenfalls gut zu erkennen ist das eine Längsachse L besitzende Vorschubrohr 3, welches mit der Vorschubstange 4 verschweißt oder in sonstiger Form verbunden ist.

Auf den freien Abschnitt des Halterohrs 2 sind in Richtung seiner Längsachse L gesehen nacheinander ein erstes Ringband 11.1, der Zwischenring 12 und ein zweites Ringband 11.2 aufgezogen.

Die Figur 3 zeigt die gleiche Situation, wobei hier allerdings das erste Ringband 11.1 und das zweite Ringband 11.2 sowie der Zwischenring 12 geschnitten dargestellt sind.

Man kann sich anhand der Figuren 2 und 3 leicht vorstellen, dass das erste Ringband 11.1 abgeschossen wird, sobald das Vorschubrohr 3 mit Hilfe der Vorschubstange 4 ein Stück weit in Richtung des Pfeils B nach rechts geschoben wird. Das Entscheidende dabei ist, dass das zweite, nach wie vor noch auf dem Halterohr 2 sitzende Ringband 11.2 auch nach dem Abschluss des ersten Ringbandes 11.1 noch ein ganzes Stück weit vom distalen Ende des Halterohrs 2 entfernt ist und daher keine Gefahr besteht, dass ungewollt auch dieses zweite Ringband 11.2 abgeschossen wird. Dies bewirkt der Zwischenring 12. Dieser macht es, anders als in den Fällen, die im Stand der Technik bekannt geworden sind und bei denen die beiden Ringbänder unmittelbar aneinander liegen, entbehrlich, dass auch das zweite Ringband zum Abschießen des ersten Ringbandes dicht an das distale Ende des Halterohrs 2 herangeschoben wird.

Die Figur 4 zeigt den erfindungsgemäßen Zwischenring 12. Es ist zweckmäßig, wenn der Zwischenring einen im Wesentlichen dem Querschnitt des Halterohrs entsprechenden, vorzugsweise kreisförmigen Querschnitt aufweist. Idealerweise sitzt der Zwischenring 12 nicht mit Spiel auf dem Halterohr 2, sondern legt sich bei seinem Aufziehen auf das Halterohr 2 mit einer gewissen Vorspannung gegen dieses an. Zu diesem Zweck ist der Innendurchmesser des Zwischenrings 12 typischerweise um 0,1 mm bis 0,5 mm, gegebenenfalls bis 0,75 mm kleiner als der Außendurchmesser des Halterohrs 2, auf das der Zwischenring 12 aufgezogen wird.

Um eine definierte Reibung zwischen dem Zwischenring 12 und dem Halterohr 2 zu erreichen, kann es zweckmäßig sein, dafür zu sorgen, dass der Zwischenring 12 nur punktförmig bzw. lokal und nicht entlang seines gesamten Innenumfangs gegen das Halterohr 2 anliegt. Um dies zu erreichen, kann der Innenring 12 mit nach innen vorspringenden Nasen 13 versehen sein. Idealerweise kommen drei Nasen zum Einsatz, die paarweise zwischen sich jeweils einen Winkel α von etwa 120° einschließen, vgl. Figur 4. Selbstverständlich kann die Anzahl der Nasen 13 auch höher sein, obwohl sich die Verwendung von nur drei Nasen als sehr vorteilhaft erwiesen hat, weil hierdurch ein leichtes Aufziehen des Zwischenrings 12 auf das Halterohr 2 ermöglicht wird.

Idealerweise ist die Breite des Zwischenrings 12 in Richtung parallel zur Längsachse L größer als die Breite oder der Durchmesser der Ringbänder parallel zur Längsachse L. Ein Verhältnis von mindestens 1,5:1 oder sogar besser noch mindestens 2:1 zu Gunsten der Breite des Zwischenrings 12 hat sich bewährt. Dieses Verhältnis ist in den Figuren nicht dargestellt.

Sofern ein Ligator zum Einsatz kommt, dessen Halterohr in der Richtung seiner Längsachse L entsprechend lang ausgestaltet ist, können selbstverständlich auch drei Ringbänder 11, die durch zwei Zwischenringe 12 voneinander getrennt sind, auf das Halterohr aufgezogen werden. Auch eine noch größere Anzahl ist theoretisch denkbar, wenn auch nicht unbedingt praktikabel.

Die Ringbänder bestehen üblicherweise aus einem gummielastischen Material, dessen Innendurchmesser typischerweise um mindestens 100 %, besser um mindestens 200 % oder sogar mehr aufgedehnt wird, wenn das Ringband auf das Halterohr 2 aufgezogen wird. Die Ringbänder haben in ihrem unverformten Zustand einen im Wesentlichen rechteckigen Querschnitt. Der Zwischenring 12 besteht hingegen aus einem andersartigen Material, bei dem es sich vorzugsweise ebenfalls um einen Kunststoff handelt, um den Zwischenring als preisgünstiges Einwegteil zur Verfügung stellen zu können. Der Innendurchmesser des Zwischenrings 12 wird aber durch das Aufziehen des Zwischenrings auf das Halterohr 2 üblicherweise nur um weniger als 10 %, meist sogar nur um weniger als 5 % aufgedehnt. Der Zwischenring 12 besteht im Regelfall aus einem Material, dessen Federhärte D um mindestens den Faktor 5, meist sogar um mindestens den Faktor 10, größer ist als die Federhärte des Materials, aus denen die Ringbänder 11 sind.

### Bezugszeichenliste

- 1: Ligator
- 2: Halterohr
- 3: Vorschubrohr
- 4: Vorschubstange
- 5: Verbindungsstück
- 6: Handstück
- 7: Abzug
- 8: Rückholfeder
- 9: Lagerblock
- 10: Verbindungslager
- 11: Ringband
- 11.1: erstes Ringband
- 11.2: zweites Ringband
- 12: Zwischenring
- 13: Nase
- L: Längsachse des Halterohrs 2
- R: Rändel
- A: Bewegungsrichtung des Abzugs 7
- B: Bewegungsrichtung des Vorschubrohrs 3

## Patentansprüche

1. Ligatorsystem zum Applizieren eines aufgeweiteten Ligatur-Rings an einen durch den Ring in seinem entspannten Zustand abzubindenden anatomischen Bereich eines Lebewesens, das einen Ligator (1) mit einem Halterohr (2) *und einem Vorschubrohr (3)* umfasst, *wobei das Halterohrs (2)* den jeweils abzubindenden anatomischen Bereich in seinem Inneren aufnehmen kann und mit seiner Außenfläche mehrere hintereinander angeordnete *Ligatur-Ringe aufgespannt halten kann,* wobei das Ligatorsystem *zusätzlich* mindestens zwei Ligatur-Ringe umfasst, **dadurch gekennzeichnet, dass** das Ligatorsystem weiterhin mindestens einen ***aus einem anderen Material als die Ligatur-Ringe bestehenden*** Zwischenring (12) umfasst, der auf dem Halterohr (2) zwischen zwei Ligatur-Ringen angeordnet ist und die beiden Ligatur-Ringe in Richtung der Längsachse (L) des Halterohrs (2) auf Abstand voneinander hält, wobei die beiden Ligatur-Ringe und der zwischen ihnen befindliche Zwischenring derart auf dem Halterohr des Ligators (1) angeordnet sind, dass sie zum Abfeuern eines der besagten Ligatur-Ringe mit Hilfe des Vorschubrohrs (3) gemeinsam das Halterohr entlang in Richtung hin zu dessen distalem Ende geschoben werden können.

2. Ligatorsystem nach Anspruch 1, **dadurch gekennzeichnet, dass** der Zwischenring (12) eine Federhärte besitzt, die mindestens um den Faktor 10 größer ist als die Federhärte eines Ligatur-Rings.

3. Ligatorsystem nach einem der vorhergehenden Ansprüche, **dadurch gekennzeichnet, dass** der Zwischenring (12), bezogen auf seine Montageposition auf dem Halterohr (2), eine Breite in Richtung der Längsachse (L) des Halterohrs (2) besitzt, die mehr als das 1,5-fache, besser mehr als das 2-fache, der Erstreckung eines Ligatur-Rings besitzt,
wenn dieser sich in seiner Montageposition auf dem Halterohr (2) befindet.

4. Ligatorsystem nach einem der vorhergehenden Ansprüche, **dadurch gekennzeichnet, dass** der Zwischenring (12) nur lokal, nämlich mit mindestens drei sich in radial einwärtiger Richtung erstreckenden Vorsprüngen, an dem Halterohr (2) anliegt.

5. Ligatorsystem nach einem der vorhergehenden Ansprüche, **dadurch gekennzeichnet, dass** der Zwischenring (12) derart an das Halterohr (2) angepasst ist, dass er beim Aufschieben auf das Halterohr (2) eine elastische Verformung erfährt und dadurch derart vorgespannt gegen die Oberfläche des Halterohrs (2) anliegt, dass er nur unter Überwindung von Reibung auf dem Halterohr (2) verschoben werden kann.

6. Ligatorsystem nach einem der vorhergehenden Ansprüche, **dadurch gekennzeichnet, dass** der kleinste Innendurchmesser des Zwischenrings (12) um 0,1 mm bis 0,5 mm kleiner ist, als der Außendurchmesser des Halterohrs (2).

7. Ligatorsystem nach einem der vorhergehenden Ansprüche, **dadurch gekennzeichnet, dass** das Ligatorsystem eine Vielzahl von Zwischenringen (12) umfasst.

8. Ligatorsystem nach Anspruch 1, **dadurch gekennzeichnet, dass** der Zwischenring (12) aus Metall besteht.

9. Ligatorsystem nach Anspruch 1, **dadurch gekennzeichnet, dass** der Zwischenring (12) aus Kunststoff besteht.

10. Ligatorsystem nach Anspruch 1, **dadurch gekennzeichnet, dass** der Zwischenring (12) nur an 3 bis 4 definiert voneinander beabstandeten Stellen gegen die Oberfläche des Halterohrs (2) anliegt.

## Claims

1. A ligator system for the application of an expanded ligature ring to a anatomical region of a living organism to be ligated by the ring in its relaxed state, which comprises a ligator (1) with a holding tube (2) and an advancing tube (3), wherein the holding tube (2) is able to accommodate in its interior the anatomical region respectively to be ligated and to keep with its outer face several ligature rings, which are disposed one behind the other, stretched wide, wherein the ligator system additionally comprises at least two ligature rings,
**characterized in that**
the ligator system further comprises at least one intermediate ring (12), which is made from a different material than the ligature rings and is disposed on the holding tube (2) between two ligature rings and keeps the two ligature rings at a distance from each other in the direction of the longitudinal axis (L) of the holding tube (2), wherein the two ligature rings and the intermediate ring located between them are disposed on the holding tube of the ligator (1) in such a way that, by means of the advancing tube (3), they can be pushed, together, along the holding tube in the direction towards the distal end thereof in order to fire one of said ligature rings.

2. The ligator system according to claim 1, **characterized in that** the intermediate ring (12) has a spring stiffness that is greater by at least the factor 10 than the spring stiffness of a ligature ring.

3. The ligator system according to any one of the preceding claims, **characterized in that** the intermediate ring (12), in relation to its mounting position on the holding tube (2), has a width in the direction of the longitudinal axis (L) of the holding tube (2) that has more than 1.5 times, better more than 2 times, the extent of a ligature ring when the latter is located at its mounting position on the holding tube (2).

4. The ligator system according to any one of the preceding claims, **characterized in that** the intermediate ring (12) rests against the holding tube (2) only locally, namely with at least three projections extending in a radially inward direction.

5. The ligator system according to any one of the preceding claims, **characterized in that** the intermediate ring (12) is adapted to the holding tube (2) in such a manner that, when it is pushed onto the holding tube (2), it undergoes an elastic deformation and thus rests against the surface of the holding tube (2) biased in such a way that it can only be displaced on the holding tube (2) by overcoming friction.

6. The ligator system according to any one of the preceding claims, **characterized in that** the smallest inner diameter of the intermediate ring (12) is smaller by at least 0.1 mm to 0.5 mm than the outer diameter of the holding tube (2).

7. The ligator system according to any one of the preceding claims, **characterized in that** the ligator system comprises a plurality of intermediate rings (12).

8. The ligator system according to claim 1, **characterized in that** the intermediate ring (12) is made from metal.

9. The ligator system according to claim 1, **characterized in that** the intermediate ring (12) is made from plastic.

10. The ligator system according to claim 1, **characterized in that** the intermediate ring (12) rests against the surface of the holding tube (2) only at 3 to 4 points spaced apart from each other in a defined manner.

## Revendications

1. Système de ligateur destiné à appliquer une bague de ligature expansée sur une zone anatomique d'un être vivant, ladite zone devant être amarrée par la bague dans son état détendu, ledit système comprenant un ligateur (1) pourvu d'un tube de maintien (2) et d'un tube d'avance (3), le tube de maintien (2) pouvant recevoir dans son volume intérieur la zone anatomique respective à amarrer et pouvant maintenir tendues plusieurs bagues de ligature, disposées les unes derrière les autres, par sa surface extérieure, le système de ligateur comprenant en supplément au moins deux bagues de ligature,
**caractérisé en ce que**
le système de ligateur comprend en outre au moins une bague intermédiaire (12) constituée d'un matériau autre que celui des bagues de ligature, laquelle est disposée sur le tube de maintien (2) entre deux bagues de ligature et maintient les deux bagues de ligature à distance l'une de l'autre en direction de l'axe longitudinal (L) du tube de maintien (2), les deux bagues de ligature et la bague intermédiaire située entre celles-ci étant disposées sur le tube de maintien du ligateur (1) de manière à pouvoir être poussées conjointement le long du tube de maintien en direction vers son extrémité distale afin de faire sauter l'une desdites bagues de ligature à l'aide du tube d'avance (3).

2. Système de ligateur selon la revendication 1, **caractérisé en ce que** la bague intermédiaire (12) possède une raideur élastique qui est supérieure au moins du facteur 10 à la raideur élastique d'une bague de ligature.

3. Système de ligateur selon l'une des revendications précédentes, **caractérisé en ce que** par référence à sa position de montage sur le tube de maintien (2), la bague intermédiaire (12) possède une largeur en direction de l'axe longitudinal (L) du tube de maintien (2), qui est de plus de 1,5 fois, et mieux plus de 2 fois l'extension d'une bague de ligature lorsque celle-ci se trouve dans sa position de montage sur le tube de maintien (2).

4. Système de ligateur selon l'une des revendications précédentes, **caractérisé en ce que** la bague intermédiaire (12) ne s'appuie que localement sur le tube de maintien (2), à savoir par au moins trois saillies qui s'étendent en direction radialement vers l'intérieur.

5. Système de ligateur selon l'une des revendications précédentes, **caractérisé en ce que** la bague intermédiaire (12) est adaptée au tube de maintien (2) de manière à subir une déformation élastique lors de l'application sur le tube de maintien (2) et à s'appuyer ainsi sous précontrainte contre la surface du tube de maintien (2) de telle sorte qu'elle ne peut être déplacée sur le tube de maintien (2) qu'en surmontant une friction.

6. Système de ligateur selon l'une des revendications précédentes, **caractérisé en ce que** le plus petit diamètre intérieur de la bague intermédiaire (12) est inférieur de 0,1 mm à 0,5 mm au diamètre extérieur du tube de maintien (2).

7. Système de ligateur selon l'une des revendications précédentes, **caractérisé en ce que** le système de ligateur comprend une multitude de bagues intermédiaires (12).

8. Système de ligateur selon la revendication 1, **caractérisé en ce que** la bague intermédiaire (12) est constituée en métal.

9. Système de ligateur selon la revendication 1, **caractérisé en ce que** la bague intermédiaire (12) est constituée en matière plastique.

10. Système de ligateur selon la revendication 1, **caractérisé en ce que** la bague intermédiaire (12) ne s'appuie sur la surface du tube de maintien (2) que sur 3 à 4 emplacements situés à une distance définie les uns des autres.
